(19) Europäisches Patentamt / European Patent Office / Office européen des brevets

(11) **EP 3 597 269 A1**

(12) **EUROPEAN PATENT APPLICATION**
published in accordance with Art. 153(4) EPC

(43) Date of publication:
**22.01.2020 Bulletin 2020/04**

(51) Int Cl.:
**A61N 5/10** *(2006.01)*

(21) Application number: **18767199.5**

(22) Date of filing: **09.03.2018**

(86) International application number:
**PCT/JP2018/009314**

(87) International publication number:
**WO 2018/168713 (20.09.2018 Gazette 2018/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.03.2017 JP 2017047185**

(71) Applicant: **Sumitomo Heavy Industries, Ltd. Tokyo 141-6025 (JP)**

(72) Inventor: **MUKAWA, Tetsuya**
**Yokosuka-shi**
**Kanagawa 237-8555 (JP)**

(74) Representative: **Louis Pöhlau Lohrentz**
**Patentanwälte**
**Postfach 30 55**
**90014 Nürnberg (DE)**

(54) **NEUTRON CAPTURE THERAPY SYSTEM AND CONTROL DEVICE**

(57) A neutron capture therapy system (100) that irradiates an object to be irradiated with a neutron beam by using an irradiation device (130), and includes a treatment planning device (110), a control device (120), and the irradiation device (130) included in a network via which transmission and reception of information are possible, in which the treatment planning device (110) creates treatment plan information related to a treatment plan and transmits the treatment plan information to the control device (120), in which the control device (120) adjusts the treatment plan information transmitted from the treatment planning device (110) to be usable in the irradiation device (130), and in which the irradiation device (130) irradiates the object to be irradiated with the neutron beam on the basis of the treatment plan information adjusted by the control device (120).

FIG. 3

EP 3 597 269 A1

**Description**

Technical Field

The present invention relates to a neutron capture therapy system and a control device.

Background Art

As a treatment method using radiation, there is neutron capture therapy (NCT) for killing cancer cells by applying neutron beams . In the neutron capture therapy, a substance such as boron incorporated in advance into a cancer cell is irradiated with neutron beams, and the cancer cell is selectively destroyed by scattering of heavy charged particles generated due to the irradiation (for example, refer to PTL 1).

Citation List

Patent Literature

[PTL 1] Specification of Japanese Patent No. 5410608

Summary of Invention

Technical Problem

In radiation therapy, as a preliminary stage of the therapy, a therapy plan is created by using a treatment planning device planning irradiation with radiation while taking into consideration the influence or the like when an affected part and a region other than the affected part are irradiated with the radiation. Treatment planning software for general radiation therapy such as X-ray therapy or photon beam therapy has been widespread, and treatment planning data created by the treatment planning software is readable by an irradiation device side. However, since treatment planning software for neutron capture therapy is not widespread, in the related art, settings of an irradiation time and the like based on a treatment plan created by treatment planning software for general radiation therapy are manually input to a neutron beam irradiation device side by an operator or the like. Thus, in a case where there is an error in an input content, irradiation with a neutron beam from a neutron beam irradiation device may not be appropriately performed.

The present invention has been made in light of the circumstances, and an object thereof is to provide a neutron capture therapy system and a control device capable of appropriately performing irradiation with a neutron beam based on a treatment plan.

Solution to Problem

In order to achieve the object, according to an aspect of the present invention, there is provided a neutron capture therapy system that irradiates an object to be irradiated with a neutron beam by using an irradiation device, the neutron capture therapy system including a treatment planning device, a control device, and the irradiation device which are included in a network via which transmission and reception of information are possible, in which the treatment planning device creates treatment plan information related to a treatment plan, in which the control device adjusts the treatment plan information created by the treatment planning device to be usable in the irradiation device, and in which the irradiation device irradiates the object to be irradiated with the neutron beam on the basis of the treatment plan information adjusted by the control device.

According to another aspect of the present invention, there is provided a control device included in a neutron capture therapy system irradiating an object to be irradiated with a neutron beam by using an irradiation device, in which the control device receives treatment plan information related to a treatment plan created and transmitted by a treatment planning device, and adjusts the treatment plan information to be usable in the irradiation device.

In the neutron capture therapy system and the control device, the control device adjusts treatment plan information created by the treatment planning device to be usable in the irradiation device. The irradiation device applies a neutron beam on the basis of the adjusted treatment plan information. The devices are included in the network via which transmission and reception of information are possible, and perform transmission and reception of information among the devices. Therefore, in the neutron capture therapy system and the control device, it is possible to appropriately perform irradiation with a neutron beam based on a treatment plan compared with a case where an operator or the like manually inputs information.

Here, the treatment plan information may include information regarding a drug concentration for each predetermined region of the object to be irradiated, and the control device may set a time for irradiating the object to be irradiated with the neutron beam on the basis of the information regarding the drug concentration for each predetermined region of the object to be irradiated.

As described above, in a case where the treatment plan information created by the treatment planning device includes the information regarding a drug concentration for each predetermined region of the object to be irradiated, and the control device calculates an irradiation time for a neutron beam in the irradiation device on the basis of the information regarding a drug concentration included in the treatment plan information, involvement of an operator in calculation of an irradiation time for a neutron beam is also unnecessary, and thus it is possible to appropriately perform irradiation with a neutron beam based on of a treatment plan.

The neutron capture therapy system may further include a drug concentration measurement device that is included in the network and acquires drug concentration information related to a drug concentration of the object to be irradiated, and the control device may recompute a time for irradiating the object to be irradiated with the neutron beam on the basis of the drug concentration information from the drug concentration measurement device.

As described above, in a case where the control device performs recomputation of an irradiation time for a neutron beam in the irradiation device on the basis of the drug concentration information transmitted from the drug concentration measurement device, it is possible to more appropriately perform irradiation with a neutron beam based on a drug concentration distribution in the body of the object to be irradiated. Involvement of an operator in recomputation of an irradiation time for a neutron beam is also unnecessary, and thus it is possible to more appropriately perform irradiation with a neutron beam.

Advantageous Effects of Invention

According to the present invention, it is possible to provide a neutron capture therapy system and a control device capable of appropriately performing irradiation with a neutron beam based on a treatment plan.

Brief Description of Drawings

Fig. 1 is a diagram for describing a summary of a neutron beam irradiation device included in a neutron capture therapy system of the present embodiment.
Fig. 2 is a diagram illustrating the periphery of a neutron beam irradiation unit of the neutron beam irradiation device in Fig. 1.
Fig. 3 is a diagram for describing each functional unit of the neutron capture therapy system.
Fig. 4 is a sequence diagram for describing a series of processes in the neutron capture therapy system.

Description of Embodiments

Hereinafter, with reference to the accompanying drawings, an embodiment of the present invention will be described. Throughout the drawings, like constituent elements are given the like reference numerals, and repeated description will be omitted.

A neutron capture therapy system according to the present embodiment is a system performing cancer treatment using neutron capture therapy, and performs cancer treatment by applying neutron beams to a position where boron is integrated in a patient to which a substance (for example, boron ($^{10}$B)) integrated into a cancer cell is administered. The neutron capture therapy system includes an irradiation device (neutron capture therapy device) irradiating a patient with neutron beams, a control device controlling the irradiation device, and a treatment planning device planning irradiation with neutron beams in the irradiation device. Prior to description of details of the system, first, a neutron beam irradiation device (neutron capture therapy device) applying a neutron beam will be described, and then the neutron capture therapy system will be described.

(Neutron Beam Irradiation Device)

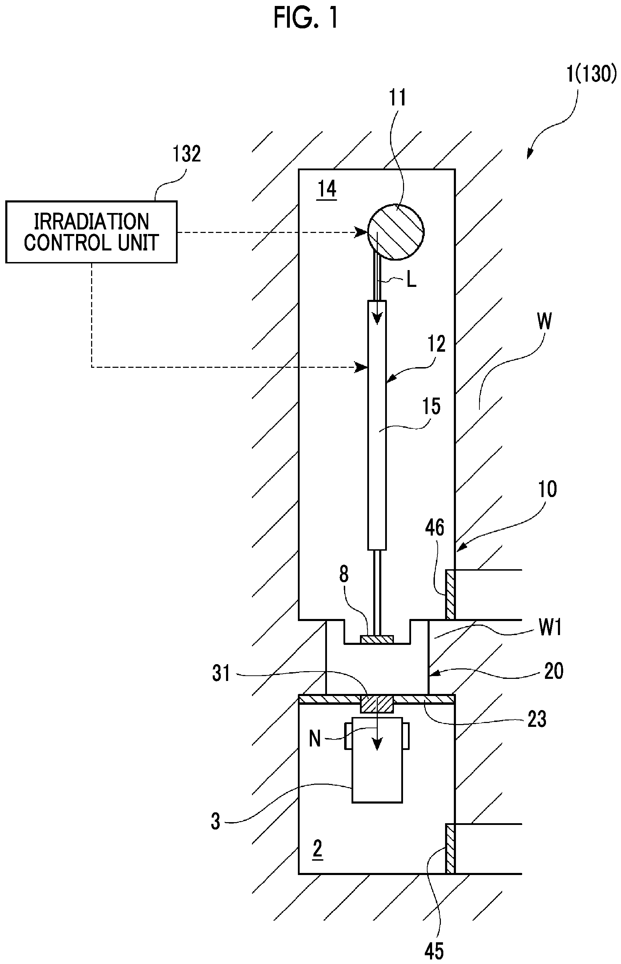

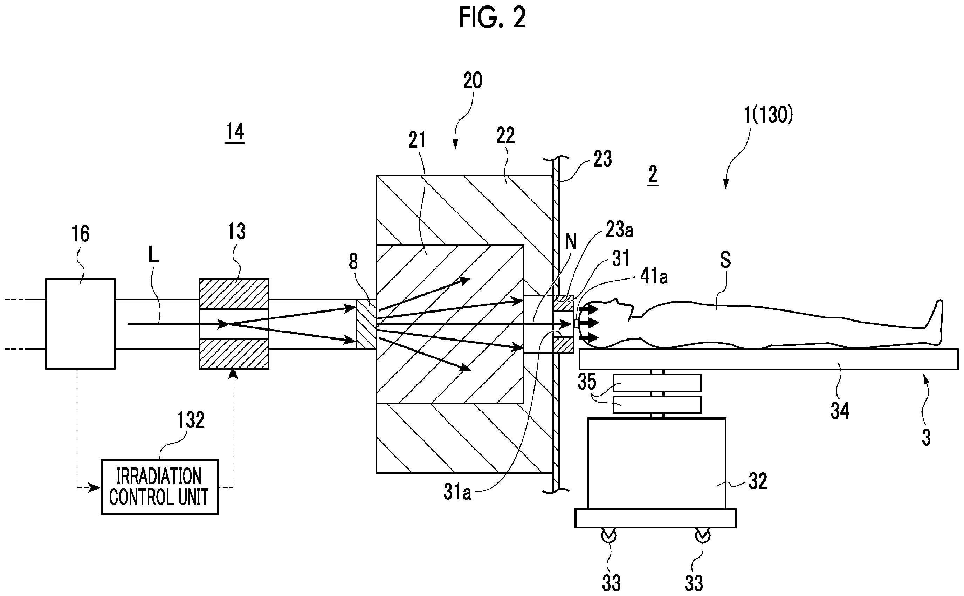

With reference to Figs. 1 and 2, a description will be made of a summary of a neutron beam irradiation device. As illustrated in Figs. 1 and 2, a neutron beam irradiation device 1 includes an irradiation room 2 in which cancer treatment of a patient S is performed by irradiating the patient (object to be irradiated) S constrained to a treatment table 3 with a neutron beam N.

Preparation work of constraining the patient S to the treatment table 3 is performed a preparation room (not illustrated) other than the irradiation room 2, and the treatment table 3 to which the patient S is constrained is moved from the preparation room to the irradiation room 2. The neutron beam irradiation device 1 includes a neutron beam generation section 10 generating the neutron beam N for treatment, and a neutron beam irradiation section 20 irradiating the patient S constrained to the treatment table 3 with the neutron beam N in the irradiation room 2. The irradiation room 2 is covered with a shield wall W, but may be provided with a passage and a door 45 through which a patient or a worker passes.

The neutron beam generation section 10 includes an accelerator 11 that accelerates charged particles so as to emit a charged particle beam L, a beam transport path 12 along which the charged particle beam L emitted from the accelerator 11 is transported, a charged particle beam irradiation unit 13 that performs irradiation with the charged particle beam L and thus controls an irradiation position of the charged particle beam L for a target 8, the target 8 that is irradiated with the charged particle beam L to cause a nuclear reaction and thus generates the neutron beam N, and a current monitor 16 that measures a current of the charged particle beam L. The accelerator 11 and the beam transport path 12 are disposed in a substantially rectangular charged particle beam generation room 14, and the charged particle beam generation room 14 is a space covered with the shield wall W made of concrete. The charged particle beam generation room 14 may be provided with a passage and a door 46 through which a worker passes to perform maintenance. The charged particle beam generation room 14 is not limited to a substantially rectangular shape, and may have other shapes. For example, in a case where a path from the accelerator to the target has an L shape, the charged particle beam generation room 14 may also have an L shape. The charged particle beam irradiation unit 13 controls, for example, an irradiation position of the charged particle beam L for the target 8, and the current monitor 16 measures a current of the charged particle beam L applied to the target 8.

The accelerator 11 accelerates charged particles and thus generates the charged particle beam L. In the present embodiment, a cyclotron is used as the accelerator 11. As the accelerator 11, instead of the cyclotron, other accelerators such as a synchrotron, a synchrocyclotron or a linac may be used.

One end (an end part on an upstream side) of the beam transport path 12 is coupled to the accelerator 11. The beam transport path 12 is provided with a beam adjustment unit 15 adjusting the charged particle beam L. The beam adjustment unit 15 has a horizontal type steering electromagnet and a horizontal-vertical type steering electromagnet adjusting an axis of the charged particle beam L, a four-pole electromagnet suppressing divergence of the charged particle beam L, and four-way slits shaping the charged particle beam L. The beam adjustment unit 15 may be omitted as long as the beam transport path 12 has a function of transporting the charged particle beam L.

The charged particle beam L transported along the beam transport path 12 is subjected to control of an irradiation position by the charged particle beam irradiation unit 13, and is applied to the target 8. The charged particle beam irradiation unit 13 may be omitted, and the charged particle beam L may be applied to an identical location of the target 8 at all times.

The target 8 is irradiated with the charged particle beam L, and thus generates the neutron beam N. The target 8 is made of, for example, beryllium (Be), lithium (Li), tantalum (Ta), or tungsten (W), and is formed in a plate shape (details of a material of the target 8 will be described later). The target 8 is not limited to a plate shape, and may be, for example, a liquid (liquid metal). The neutron beam N generated by the target 8 is applied toward the patient S in the irradiation room 2 by the neutron beam irradiation section 20.

The neutron beam irradiation section 20 includes a deceleration member 21 that decelerates the neutron beam N emitted from the target 8, and a shield member 22 that blocks radiation such as the neutron beam N and gamma rays from being discharged to the outside, and the deceleration member 21 and the shield member 22 configure a moderator.

The deceleration member 21 has a laminated structure made of, for example, a plurality of different materials, and materials of the deceleration member 21 are selected as appropriate according to conditions such as energy of the charged particle beam L. Specifically, for example, in a case where a photon beam of 30 MeV is output from the accelerator 11, and a beryllium target is used as the target 8, materials of the deceleration member 21 may be lead, iron, and aluminum or calcium fluoride.

The shield member 22 is provided to surround the deceleration member 21, and has a function of blocking radiation such as the neutron beam N and gamma rays due to generation of the neutron beam N from being discharged to the outside of the shield member 22. At least a part of the shield member 22 may be buried in a wall W1 separating the charged particle beam generation room 14 from the irradiation room 2, and may not be buried therein. A wall body 23 forming a part of a lateral wall surface of the irradiation room 2 is provided between the irradiation room 2 and the shield member 22. The wall body 23 is provided with a collimator attachment portion 23a serving as an output port of the neutron beam N. A collimator 31 defining an irradiation field of the neutron beam N is fixed to the collimator attachment portion 23a. The collimator 31 may be attached to the treatment table 3 which will be described later instead of the collimator attachment portion 23a being provided at the wall body 23.

In the neutron beam irradiation section 20, the charged particle beam L is applied to the target 8, and thus the target 8 generates the neutron beam N. The neutron beam N generated by the target 8 is decelerated when passing through the deceleration member 21, and the neutron beam N emitted from the deceleration member 21 passes through the collimator 31 and is applied to the patient S on the treatment table 3. Here, as the neutron beam N, a thermal neutron beam or an epithermal neutron beam having relatively low energy may be used.

The treatment table 3 functions as a mounting table used for neutron capture therapy, and is movable to the irradiation room 2 from the preparation room (not illustrated) in a state of being mounted with the patient S. The treatment table 3 includes a base portion 32 forming a base of the treatment table 3, casters 33 that enable the base portion 32 to be moved on a floor, a top plate 34 mounting the patient S thereon, and drive portions 35 moving the top plate 34 relative to the base portion 32. The base portion 32 may be fixed to the floor without using the casters 33.

The neutron beam irradiation device 1 includes an irradiation control unit 132 performing various processes. The irradiation control unit 132 is electrically coupled to the accelerator 11, the beam adjustment unit 15, the charged particle beam irradiation unit 13, and the current monitor 16. The accelerator 11, the beam adjustment unit 15, and the charged particle beam irradiation unit 13 are controlled on the basis of adjusted treatment plan information transmitted from the control device which will be described later, and a detection result output from the current monitor 16.

(Neutron Capture Therapy System)

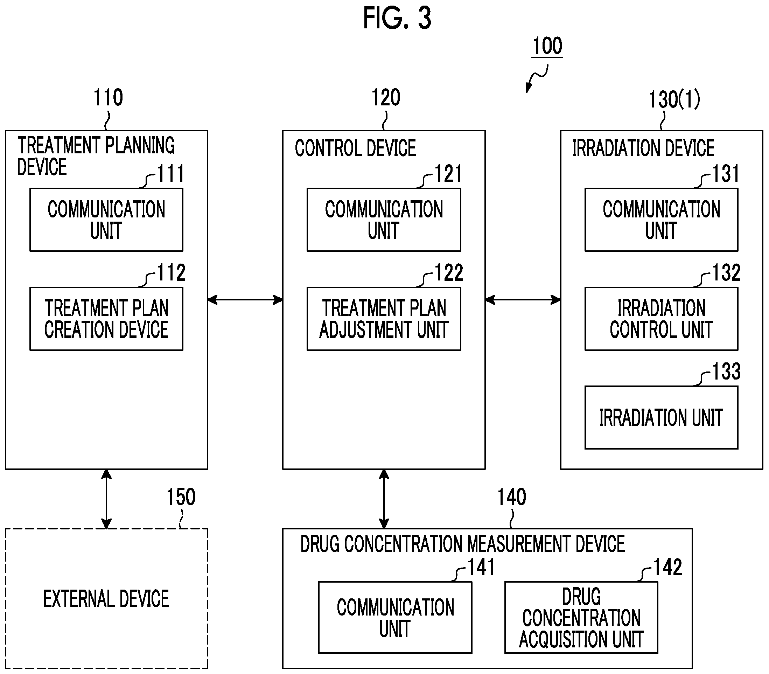

Next, the neutron capture therapy system including the neutron beam irradiation device 1 will be described with reference to Fig. 3.

As illustrated in Fig. 3, a neutron capture therapy system 100 includes a treatment planning device 110, a control device 120, an irradiation device 130, and a drug concentration measurement device 140. The treatment planning device 110 is a device making a treatment plan for neutron capture therapy for irradiating the patient S with neutrons. The control device 120 is a device controlling the irradiation device 130 on the basis of treatment plan information created by the treatment planning device 110. The irradiation device 130 is a device irradiating the patient S with neutron beams on the basis of an instruction from the control device 120. The neutron beam irradiation device 1 as illustrated in Figs. 1 and 2 corresponds to the irradiation device 130 of the neutron capture therapy system 100. The drug concentration measurement device 140 is a device measuring a drug concentration of the patient S on the day on which irradiation with neutron beams is performed by the irradiation device 130. The drug concentration is a boron concentration of the patient S in boron neutron capture therapy, and a drug concentration distribution may be obtained on the basis of, for example, a ratio between a boron concentration in blood and a boron concentration of each tissue in the body of the patient S. Alternatively, a drug concentration of the patient S may be measured by using prompt gamma-ray single photon emission computed tomography (PG-SPECT) device or a positron emission tomography (PET) device. The drug concentration differs for each region (for example, for each tissue) that is a neutron beam irradiation target even in the body of the patient S. In the neutron capture therapy, an effect of neutron beam irradiation changes according to the concentration of a drug accumulated in the body of the patient S. Therefore, a drug concentration of each predetermined region in the body of the patient S greatly influences an amount of neutron beams to be applied.

The treatment planning device 110, the control device 120, the irradiation device 130, and the drug concentration measurement device 140 included in the neutron capture therapy system 100 are connected to each other via a wired or wireless network, and can thus transmit and receive information to and from each other.

Each of the treatment planning device 110, the control device 120, the irradiation device 130, and the drug concentration measurement device 140 is physically configured to include a computer system including a CPU, a RAM and a ROM that are main storage devices, a communication module that is a data transmission/reception device, an auxiliary storage device such as a hard disk or a flash memory, an input device such as a keyboard, and an output device such as a display. In the treatment planning device 110, the control device 120, the irradiation device 130, and the drug concentration measurement device 140, predetermined computer software is read on hardware such as the CPU or the RAM such that the communication module, the input device, and the output device are operated under the control of the CPU, and reading and writing of data are performed on the RAM or the auxiliary storage device such that a series of functions in the respective devices is realized.

A description will be further made of functions of each device configuring the neutron capture therapy system 100. First, the treatment planning device 110 includes a communication unit 111 and a treatment plan creation device 112 .

The communication unit 111 has a function of transmitting information regarding a treatment plan created by the treatment plan creation device 112 to the control device 120. The treatment plan creation device 112 has a function of creating a treatment plan regarding application of neutron beams to the patient S. The information (treatment plan information) regarding the treatment plan created by the treatment plan creation device 112 is transmitted from the treatment planning device 110 to the control device 120 by the communication unit 111.

Creation of a treatment plan is performed, for example, according to the following procedures. First, the treatment plan creation device 112 acquires an image of the patient S, sets a contour of the body of the patient S, and then sets a region of a tissue (a bone, an organ, or the like) of the patient S for each tissue. Thereafter, a dose of neutron beams applied to the patient S is set, and a dose distribution in a case where neutron beams with the dose are applied to the patient S is calculated, on the basis of the concentration of a drug in the body of the patient S, an atomic composition corresponding to each tissue, and the resistance to a neutron beam. In other words, in the treatment plan creation device 112, information regarding a three-dimensional dose distribution when neutron beams are applied to the patient S is created as a treatment plan. The treatment plan created by the treatment plan creation device 112 includes, as the information regarding three-dimensional dose distribution in the body of the patient S, a dose upper limit when a neutron beam is applied, a dose rate based on administration of a drug, a dose rate derived from a tissue, and information regarding a drug concentration in the body of the patient S, for each "grid" indicating a region used as a basic unit for neutron beam irradiation. In creation of a treatment plan in the treatment planning device 110, for example, information from an external device 150 such as a CT device may be used.

The control device 120 includes a communication unit 121 and a treatment plan adjustment unit 122.

The communication unit 121 has a function of receiving the treatment plan information created and transmitted by the treatment planning device 110 and drug concentration information created and transmitted by the drug concentration measurement device 140 which will be described later, and transmitting adjusted treatment plan information that is adjusted by the treatment plan adjustment unit 122 to the irradiation device 130.

The treatment plan adjustment unit 122 has a function of adjusting the treatment plan information created by the treatment planning device 110 on the basis of the drug concentration information created and transmitted by the drug concentration measurement device 140 which will be described later.

Adjustment of a treatment plan indicates that the treatment plan information created by the treatment planning device 110 is adjusted to be usable in the irradiation device 130 (neutron beam irradiation device 1). The treatment plan information includes information regarding a dose distribution in the body of the patient S, but, in the irradiation device 130, it is necessary to set a neutron beam irradiation time (neutron beam irradiation amount) for each predetermined region (for example, for each grid) in the body of the patient S, and to apply a neutron beam according to the setting. Therefore, the control device 120 sets conditions (an irradiation time (neutron beam irradiation amount) for each irradiation region) that are necessary when a neutron beam is applied in the irradiation device 130. This process is referred to as "adjustment of a treatment plan". The adjustment of a treatment plan includes, for example, adjusting a format to be usable in the irradiation device 130.

In the neutron capture therapy, it is general to recalculate and set the time for irradiating the patient S with a neutron beam on the basis of information (drug concentration information) regarding a drug concentration on the day of treatment of the patient S. Therefore, the adjustment of a treatment plan in the treatment plan adjustment unit 122 also includes correction of an irradiation time based on the drug concentration information related to the patient S. The treatment plan adjustment unit 122 has a function of performing the correction of a treatment plan.

The irradiation device 130 includes a communication unit 131, an irradiation control unit 132, an irradiation unit 133.

The communication unit 131 has a function of receiving adjusted treatment plan information that is adjusted and transmitted by the control device 120. The irradiation control unit 132 has a function of controlling the irradiation unit 133 of the irradiation device 130 on the basis of the adjusted treatment plan information.

The irradiation unit 133 has a function of irradiating the patient S with neutron beams on the basis of the adjusted treatment plan information under the control of the irradiation control unit 132. The irradiation unit 133 of the irradiation device 130 includes the neutron beam generation section 10 generating the neutron beam N for treatment, and the neutron beam irradiation section 20 irradiating the patient S constrained to the treatment table 3 with the neutron beam N in the irradiation room 2, in the neutron beam irradiation device 1 described in Figs. 1 and 2.

The drug concentration measurement device 140 includes a communication unit 141 and a drug concentration acquisition unit 142.

The communication unit 141 has a function of transmitting drug concentration information that is information regarding a blood drug concentration of the patient S acquired and created by the drug concentration acquisition unit 142, to the control device 120.

The drug concentration acquisition unit 142 has a function of acquiring information regarding blood drug concentration of the patient S, and creating drug concentration information to be transmitted to the control device 120. The drug concentration acquisition unit 142 may have a function of measuring a drug concentration of the patient S, and may have only a function of acquiring information regarding a drug concentration measured by another device or the like, and transmitting the drug concentration information to the control device 120. A drug concentration may be measured by using, for example, a single photon emission computed tomography (SPECT) device, a high-frequency inductively coupled plasma (ICP) device, or a prompt gamma-ray detection device. In other words, the drug concentration measurement device 140 may be configured to include such a device.

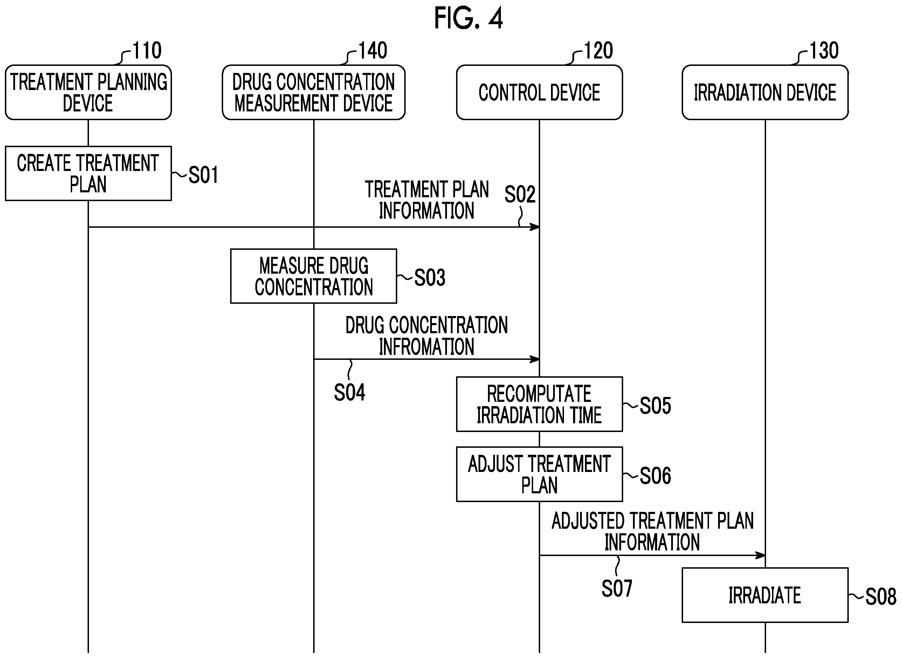

Next, with reference to Fig. 4, a description will be made of a series of processes in the neutron capture therapy system 100.

First, the treatment plan creation device 112 of the treatment planning device 110 creates a treatment plan related to neutron capture therapy for the patient S (S01). The treatment plan information created by the treatment plan creation device 112 is transmitted to the control device 120 via the communication unit 111, and is received by the communication unit 121 of the control device 120 (S02).

On the other hand, in the drug concentration measurement device 140, the drug concentration acquisition unit 142 acquires drug concentration information related to the patient S (S03), and the drug concentration information is transmitted from the communication unit 141 to the control device 120, and is received by the communication unit 121 of the control device 120 (S04) . Since a drug concentration on the day of treatment of the patient S is used as the drug concentration information, the according to of the drug concentration information (S03) and the transmission of the drug concentration information (S04) in the drug concentration measurement device 140 are performed on the day of treatment of the patient S.

Next, the treatment plan adjustment unit 122 of the control device 120 performs recomputation of an irradiation time (S05) and adjustment of the treatment plan (S06) on the basis of the treatment plan information from the treatment planning device 110 and the drug concentration information from the drug concentration measurement device 140.

A description will be made of a method of the recomputation of an irradiation time (S05) performed by the treatment plan adjustment unit 122. The treatment plan information includes a three-dimensional dose distribution in a case where neutron beams are applied for an irradiation time obtained for the patient S in advance. Therefore, in the recomputation of an irradiation time, the following computation for a region (grid) for which a dose is calculated is performed, a neutron beam irradiation time for which a dose reaches an upper limit (an upper limit of a dose) in each grid, and the minimum value thereof is set as a treatment irradiation time.

Specifically, in a case where a time rate [second] to reach a dose upper limit when a neutron beam is applied in each grid is indicated by $T_{grid}$, $T_{grid}$ may be acquired according to the following Equation (1).

$$T_{grid} = \frac{D_{limit}}{Dr_{10_B} \times \left( N_{10_{B(irr)}} \middle/ N_{10_{B(plan)}} \right) + Dr_{tissue}} \tag{1}$$

$D_{limit}$ indicates a dose upper limit [Gy] in the grid, and is set for each tissue (for each normal tissue or affected tissue, for each organ, or for each grid) . $D_{\gamma 10B}$ indicates a dose rate [Gy/s] given by administration in each grid when a treatment plan is created, and is set for each grid. Drtissue includes dose application caused by reaction between in-vivo nuclei and neutron beams and dose application caused by gamma rays, and indicates a dose rate [Gy/s] given due to biotissue in each grid when a treatment plan is created, and is set for each grid. $N_{10B(plan)}$ indicates a drug concentration [ppm] in each grid when a treatment plan is created, and is set on the basis of a measured value or a supposed value. $N_{10B(plan)}$ may be set for each tissue, and may be for each grid, more specifically. $D_{limit}$, $Dr_{10B}$, $Dr_{tissue}$, and $N_{10B(plan)}$ are information used during creation of a treatment plan, and are thus included in the treatment plan information.

$N_{10B(irr)}$ indicates a drug concentration [ppm] of the patient S when a neutron beam is irradiated, and is information from the drug concentration measurement device 140 to the control device 120.

In other words, the treatment plan adjustment unit 122 of the control device 120 recomputes an irradiation time by combining $D_{limit}$, $Dr_{10B}$, $Dr_{tissue}$, and $N_{10B(plan)}$ included in the treatment plan information transmitted from the treatment planning device 110 with $N_{10B(irr)}$ included in the drug concentration information transmitted from the drug concentration measurement device 140.

As a method of recomputing an irradiation time in the control device 120, methods other than the method may be used. A method of recomputation may be changed as appropriate on the basis of an irradiation condition or the like.

The treatment plan adjustment unit 122 of the control device 120 may change a format such that irradiation with a neutron beam is appropriately performed in the irradiation device 130 as the adjustment of the treatment plan (S06).

Adjusted treatment plan information obtained after the recomputation of an irradiation time (S05) and the adjustment of the treatment plan (S06) in the treatment plan adjustment unit 122 of the control device 120 is transmitted from the communication unit 121 of the control device 120, and is received by the communication unit 131 of the irradiation device 130 (S07) . In the irradiation device 130, the irradiation control unit 132 performs various pieces of control related to irradiation with a neutron beam on the basis of the adjusted treatment plan information received by the communication unit 131, and, as a result, the irradiation unit 133 of the irradiation device 130 irradiates the patient S with a neutron beam (S08).

As mentioned above, in the neutron capture therapy system 100 according to the present embodiment, treatment plan information created by the treatment planning device 110 is transmitted to the control device 120, and is then adjusted by the control device 120 such that irradiation with a neutron beam is possible in the irradiation device 130. The adjusted treatment plan information is transmitted from the control device 120 to the irradiation device 130, and the irradiation device 130 applies a neutron beam on the basis of the adjusted treatment plan information. Therefore, in the neutron capture therapy system 100 according to the present embodiment, it is possible to appropriately apply a neutron beam based on a treatment plan.

In the related art, a treatment planning device creating a treatment plan and an irradiation device irradiating the patient S with a neutron beam are provided separately from each other. Therefore, in neutron capture therapy of the related art, generally, an operator operating the irradiation device manually inputs information required for irradiation with a neutron beam on the basis of a treatment plan created by the treatment planning device. However, in a case where an operator manually controls the irradiation device on the basis of a treatment plan, a wrong operation or wrong input of the operator may occur. Therefore, irradiation with a neutron beam may not be appropriately performed based on a treatment plan.

In contrast, in the neutron capture therapy system 100 according to the present embodiment and the control device 120 included in the system, treatment plan information created in the treatment planning device 110 is transmitted to the control device 120 from the treatment planning device 110. The treatment plan is adjusted in the control device 120, adjusted treatment plan information is transmitted to the irradiation device 130 from the control device 120, and a neutron beam is applied on the basis of the adjusted treatment plan information in the irradiation device 130. Therefore, since it is possible to prevent an operator operating the irradiation device from manually inputting information required for irradiation with a neutron beam as in the procedures in the neutron capture therapy of the related art, it is possible to prevent a wrong operation caused by wrong input or the like and thus to appropriately perform irradiation with a neutron beam based on a treatment plan. In the control device 120, treatment plan information created by the treatment planning device 110 is adjusted to correspond to control of irradiation with a neutron beam in the irradiation device 130, and thus involvement of an operator in adjustment of the treatment plan information is not necessary. Therefore, it is possible to appropriately perform irradiation with a neutron beam based on of a treatment plan.

Treatment plan information created by the treatment planning device 110 includes information regarding a drug concentration for each predetermined region (for example, for each grid or for each tissue) of a patient, and the control device 120 calculates an irradiation time for a neutron beam in the irradiation device 130 on the basis of the information regarding a drug concentration included in the treatment plan information. Therefore, involvement of an operator in calculation of an irradiation time for a neutron beam is also unnecessary, and thus it is possible to appropriately perform irradiation with a neutron beam based on of a treatment plan.

In the neutron capture therapy system 100 of the embodiment, the control device 120 performs recomputation of an irradiation time for a neutron beam in the irradiation device 130 on the basis of drug concentration information transmitted from the drug concentration measurement device 140. With this configuration, it is possible to perform irradiation with a neutron beam on the basis of a drug concentration distribution in the body of the patient S, and thus it is possible to more appropriately perform irradiation with a neutron beam based on of a treatment plan. Involvement of an operator in recomputation of an irradiation time for a neutron beam in the irradiation device 130 is also unnecessary, and thus it is possible to further reduce a risk such as wrong computation.

As mentioned above, the neutron capture therapy system 100 according to one embodiment of the present invention has been described, but is not limited to the embodiment, and may be modified or applied to other embodiments within the scope without changing the concept disclosed in each claim.

For example, in the embodiment, a description has been made of a case where the treatment planning device 110, the control device 120, the irradiation device 130, and the drug concentration measurement device 140 are separately provided, but each device may be configured with a plurality of computers. Functions of two or more devices may be configured to be realized by a single device.

A description has been made of a configuration in which the drug concentration measurement device 140 is included in the neutron capture therapy system 100, but the drug concentration measurement device 140 may not be included therein. In this case, the control device 120 may not perform recomputation of an irradiation time based on drug concentration information.

In the embodiment, a description has been made of a configuration in which neutrons are generated by irradiating the target 8 with charged particle beams emitted from the accelerator, but a device configuration of a device generating and applying neutrons may be changed as appropriate. For example, there may be a device generating neutrons by using a reactor.

Reference Signs List

100 NEUTRON CAPTURE THERAPY SYSTEM
110 TREATMENT PLANNING DEVICE
120 CONTROL DEVICE
130 IRRADIATION DEVICE
140 DRUG CONCENTRATION MEASUREMENT DEVICE

**Claims**

1. A neutron capture therapy system that irradiates an object to be irradiated with a neutron beam by using an irradiation device, the neutron capture therapy system comprising:

   a treatment planning device, a control device, and the irradiation device which are included in a network via which transmission and reception of information are possible,
   wherein the treatment planning device creates treatment plan information related to a treatment plan,
   wherein the control device adjusts the treatment plan information created by the treatment planning device to be usable in the irradiation device, and
   wherein the irradiation device irradiates the object to be irradiated with the neutron beam on the basis of the treatment plan information adjusted by the control device.

2. The neutron capture therapy system according to claim 1,
   wherein the treatment plan information includes information regarding a drug concentration for each predetermined region of the object to be irradiated, and
   wherein the control device sets a time for irradiating the object to be irradiated with the neutron beam on the basis of the information regarding the drug concentration for each predetermined region of the object to be irradiated.

3. The neutron capture therapy system according to claim 2, further comprising:

   a drug concentration measurement device that is included in the network and acquires drug concentration information related to a drug concentration of the object to be irradiated,
   wherein the control device recomputes a time for irradiating the object to be irradiated with the neutron beam on the basis of the drug concentration information from the drug concentration measurement device.

4. A control device included in a neutron capture therapy system irradiating an object to be irradiated with a neutron beam by using an irradiation device, wherein the control device
   receives treatment plan information related to a treatment plan created and transmitted by a treatment planning device, and adjusts the treatment plan information to be usable in the irradiation device.

# FIG. 1

1(130)

11

132

IRRADIATION
CONTROL UNIT

14

L

W

12

15

10

46

8

W1

31

20

23

N

3

2

45

FIG. 2

EP 3 597 269 A1

FIG. 3

EP 3 597 269 A1

FIG. 4

130 IRRADIATION DEVICE

120 CONTROL DEVICE

140 DRUG CONCENTRATION MEASUREMENT DEVICE

110 TREATMENT PLANNING DEVICE

CREATE TREATMENT PLAN — S01

TREATMENT PLAN INFORMATION — S02

MEASURE DRUG CONCENTRATION — S03

DRUG CONCENTRATION INFROMATION — S04

RECOMPUTATE IRRADIATION TIME — S05

ADJUST TREATMENT PLAN — S06

ADJUSTED TREATMENT PLAN INFORMATION — S07

IRRADIATE — S08

## INTERNATIONAL SEARCH REPORT

| International application No. |
| --- |
| PCT/JP2018/009314 |

**A. CLASSIFICATION OF SUBJECT MATTER**
Int.Cl. A61N5/10(2006.01)i

According to International Patent Classification (IPC) or to both national classification and IPC

**B. FIELDS SEARCHED**

Minimum documentation searched (classification system followed by classification symbols)
Int.Cl. A61N5/10

| Documentation searched other than minimum documentation to the extent that such documents are included in the fields searched | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2016-77812 A (SUMITOMO HEAVY INDUSTRIES, LTD.) 16 May | 1, 4 |
| Y | 2016, paragraphs [0008]-[0046], fig. 1-2 & CN 105938731 A & TW 201634075 A | 2-3 |
| Y | JP 2016-159107 A (SUMITOMO HEAVY INDUSTRIES, LTD.) 05 September 2016, paragraphs [0007]-[0050], fig. 1-2 (Family: none) | 2-3 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 15 May 2018 (15.05.2018) | 05 June 2018 (05.06.2018) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**EP 3 597 269 A1**

**INTERNATIONAL SEARCH REPORT**

| International application No. |
| --- |
| PCT/JP2018/009314 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2016-214760 A (TOSHIBA CORP.) 22 December 2016, entire text, all drawings (Family: none) | 1-4 |
| A | 櫻井英幸, ＂筑波大学の加速器ベース BNCT プロジェクト＂, [online], 08 November 2016, [retrieval date 15 May 2018], internet:<URL:http://www.pref.osaka.lg.jp/attach/28090/00222649/bnctsymposiryouall-12tsukuba.pdf>, p. 66, non-official translation (SAKURAI, Hideyuki, "Accelerator based-BNCT project of University of Tsukuba") | 1-4 |
| A | TAKADA, Kenta, et al., "Development of Monte Carlo based real-time treatment planning system with fast calculation algorithm for boron neutron capture therapy", Physica Medica, 2016, vol. 32, no. 12, pp. 1846-1851 | 1-4 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5410608 B